# EUROPEAN PATENT SPECIFICATION

(11) **EP 2 845 553 B1**
(45) Date of publication and mention of the grant of the patent: **11.05.2016**
(21) Application number: 13183246.1
(22) Date of filing: 05.09.2013
(51) Int. Cl.: A61B 17/86, A61B 17/72, A61B 17/74, A61B 17/84, A61B 17/00

(54) **Bone anchor**
Knochenanker
Ancrage osseux

(43) Date of publication of application: 11.03.2015
(73) Proprietor: Biedermann Technologies GmbH & Co. KG, 78166 Donaueschingen (DE)
(72) Inventor: Biedermann, Lutz, 78048 VS-Villingen (DE)
(74) Representative: Prüfer & Partner mbB Patentanwälte · Rechtsanwälte

(56) References cited:
- EP-A2- 1 016 382
- DE-A1-102011 106 653
- FR-A1- 2 859 904
- US-A1- 2006 079 895

## Description

The present invention relates to a bone anchor for use in clinical surgery, for example in the treatment of traumatic fractures caused by osteoporosis of bones, among others. The bone anchor has a main body with a head and a shank. One or more recesses extend through the head and along a portion of the shank. The one or more recesses may extend generally parallel with respect to a longitudinal axis of the main body, and receives each a pin-shaped element to support and improve the anchoring stability of the bone anchor.

Bone anchoring assemblies comprising a bone anchor and one or more associated pins may help in preventing loosening of the bone anchors, when the pins are mounted to the anchors and extend, for example, into the surrounding bone material in an inclined fashion with respect to a longitudinal axis of the bone anchor. However, even if the associated pins extend parallel to the implanted bone anchor, a rotational support may be provided, since an unscrewing movement of the bone anchor is inhibited.

One example is disclosed in US 4,657,001. A lag screw is implanted into a femoral head, and a pin antirotation-locking assembly comprising four elongated pins connected with each other via a head is attached to the lag screw. The pins of the assembly may each slide into a respective groove provided along the entire length of the lag screw including its head portion. The pins have tips, which slope up and away from a longitudinal center line causing the pins to lift slightly out of the grooves when being driven into the bone. The pins serve to positively lock the lag screw.

A locking screw for an intramedullary nail having a head including a passage is disclosed in US 2006/0064095 A1. A longitudinal wedging element can be inserted through the passage of the head thereby extending substantially parallel fo a central longitudinal axis of the screw and along a flat ramp recessed from the screw shaft. In this case, the wedging element wedges the shaft of the locking screw in a transverse bore hole which is formed in the intramedullary nail.

Document US 2008/0262497 A1 discloses a medical device for treating fractures at the femoral head. The device has an outer tube provided with recesses and an inner tube that is connected with a distal end piece via two strips whose position upon insertion of the inner tube into the outer tube corresponds to the position of the recesses. Using a screw to advance the inner tube towards the distal end piece the two strips expand through the recesses of the outer tube and into the surrounding bone material. This bending prevents the device from loosening from the bone material in an axial direction.

Document US 2009/0204216 A1 discloses an expandable implant for stabilizing the vertebrae or bones. The implant functions like a stent and has a flexible tubular section extending between first and second ends, and by decreasing the distance between both ends, a plurality of strips of the flexible tubular section expand radially outwards pushing aside cancellous bone material and thereby stabilizing an osteoporotic vertebrae body.

Document DE 10 2011 106 653 A1 discloses an anchoring element comprising a shaft and one or more components which are wire-shaped and consist of a shape memory material or a superelastic material. Due to the shape memory effect or its superelastic properties the wire-shaped component(s) can adopt two states. The shaft has one or more recesses. Each wire-shaped component is arranged completely within one of the recesses, if the material the component is made of is in its first state. After the material's transition to the second state, a portion of the component protrudes from the recess.

It is an object to improve the anchoring stability and support a bone anchor when being implanted in particularly in osteoporotic, cancellous, or fractured bony material.

The object is solved by a bone anchor according to claim 1. Advantageous embodiments are defined in the appended claims.

According to the invention, a bone anchor is provided with a main body including a head and a shank. A recess extends through at least a portion of the head and along at least a portion of the shank, and the recess is configured to receive a pin-shaped element.

A first end of the recess provided at the head may for example be represented by an opening which allows inserting therethrough the pin-shaped element. A second end of the recess provided at the shank is formed as a stop. The stop is configured to be abutted or engaged by a distal end portion of the pin-shaped element. A locking structure is provided at the first end of the recess and exerts a biasing force on a proximal end portion of the pin-shaped element in a direction of the longitudinal axis and towards the stop. The stop exerts a counterforce and as a consequence, the pin-shaped element is compressed in the longitudinal direction. The pin-shaped element, however, has few axial compressibility but a sufficient degree of bending flexibility, and thus its intermediate bends radially outwards away from the shank upon receiving compressing forces.

According to an embodiment, the recess may include a groove portion, which is open towards the outside, i.e., towards the surrounding bony material, when the bone anchor is implanted. The groove portion may be located at the shank, but may also be located at the head. The compressed pin-shaped element may thus bend with an intermediate portion between the two end portions thereof towards the outside through the open groove portion, while it is held at the end portions. The intermediate portion bending outside thereby expands into the bony material thereby improving the anchoring stability of the implanted bone anchor. In particular, loosening by rotational movement may be inhibited.

It is not necessary that the intermediate portion of the pin-shaped element bends and expands through the open groove portion. Alternatively the recess may comprise two or more non-contiguous parts, and the pin-shaped element inserted into the multiple portion recess bends and expands radially outward in a free section extending between the end portions of the recess. For example, a portion of the shank or head of the bone anchor, for example an extended neck portion, may be configured to be considerably thinned between a through hole formed at the head and a bore hole at the distal end of the recess.

It is also possible that the groove portion is closed by a thin or weak material in a state of assembly of the parts, and breaks up only when a compressive force is exerted on the pin-shaped element inserted therein.

Embodiments provided in the detailed explanation below provide for bone anchors having each two recesses and respective pin-shaped elements on opposite sides thereof. However, it is contemplated that bone anchors according to the invention may also comprise one, two, three, four, or even more recesses and pin-shaped elements respectively. Nevertheless, a bone anchor having two recesses and assembled with two pin-shaped elements is preferred due to the symmetry and the lesser number of parts.

The locking structure which provides for the biasing force for compressing the pin-shaped element may be embodied by different mechanisms and the examples provided below are purely illustrative and do not limit the scope of the invention. Examples provided herein refer to a bayonet catch, an undercut recess, and a locking cap, respectively. Other locking mechanisms, which maintain the biasing force being exerted, are possible as well. It is noted that the locking structure as defined herein provides for maintaining the biasing force. The initiation of the biasing force, however, will have to be effected by an external tool that is not part of the claimed bone anchor.

A bone anchoring assembly, not forming part of the present invention, comprises a bone anchor having a main body including a head and a shank. A recess extending extends through at least a portion of the head and along at least a portion of the shank, and the recess is configured to receive a pin-shaped element. The pin-shaped element is made of a material that has shape memory properties, such as a shape memory alloy, for example a nickel titanium alloy such as Nitinol. The pin-shaped element is configured to assume a first configuration at a first temperature in which it is insertable into the recess and a second configuration at a second temperature different from the first temperature in which an intermediate portion of the pin-shaped element is bent in a transverse direction away from the shank. The bone anchoring assembly is inserted into the bone in the first configuration and, by changing the temperature, the pin-shaped element transforms into the second configuration whereby its intermediate portion is bent outward in a transverse direction away from the shank. The bone anchoring assembly does not require the aid of a mechanical locking structure.

Further advantages will become apparent from the following detailed description of embodiments taken in conjunction with the accompanying drawings, wherein:
- Fig. 1: shows a perspective view of a bone anchor assembly with bone anchor and pin-shaped element according to a first embodiment;
- Fig. 2: shows a cross-sectional profile of the bone anchor and pin-shaped element of Fig. 1 according to the first embodiment;
- Fig. 3: shows an enlarged perspective view of the head portion of the bone anchor of Fig. 1 according to the first embodiment;
- Fig. 4: shows a top view of the head portion of the bone anchor of Fig. 1 according to the first embodiment;
- Fig. 5: shows a cross-sectional profile of the head portion taken along a line AA shown in Fig. 4 according to the first embodiment;
- Fig. 6: shows a cross-sectional profile of the head portion taken along a line BB shown in Fig. 4 according to the first embodiment;
- Fig. 7A: shows a perspective view of the head portion with inserted pin-shaped elements during a first step of assembly according to the first embodiment;
- Fig. 7B: same as Fig. 7A, but for the complete bone anchor;
- Fig. 7C: same as Fig. 7B, but in cross-sectional profile;
- Fig. 8A: shows a perspective view of the head portion with inserted pin-shaped elements during a second step of assembly according to the first embodiment;
- Fig. 8B: same as Fig. 8A, but for the complete anchor;
- Fig. 8C: same as Fig. 8B, but in cross-sectional profile;
- Fig. 9: shows a perspective view of a bone anchor assembly with bone anchor and pin-shaped element according to a second embodiment;
- Fig. 10: shows a cross-sectional profile of the bone anchor and pin-shaped element of Fig. 9 along with a top view of the head portion according to the second embodiment;
- Fig. 11A: shows in an enlarged cross sectional view of the head portion of the bone anchor of Fig. 9 a first step of assembling the bone anchor with pin-shaped elements according to the second embodiment;
- Fig. 11B: shows in an enlarged-cross sectional view of the head portion of the bone anchor of Fig. 9 a second step of assembling the bone anchor with pin-shaped elements according to the second embodiment;
- Fig. 12A: shows a perspective view of the bone anchoring assembly in a state with expanded pin-shaped elements according to the second embodiment;

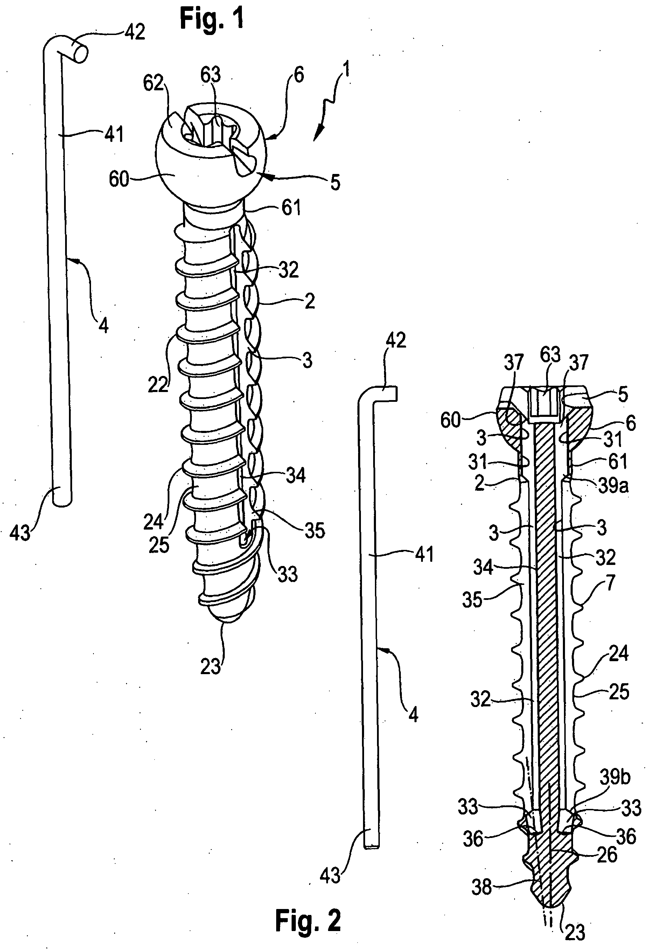

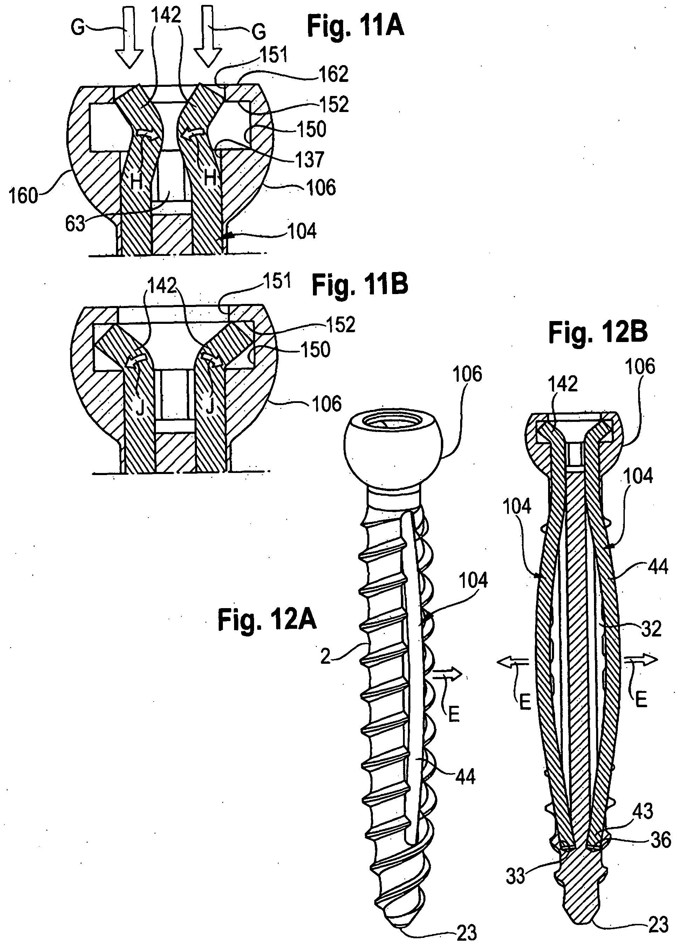

- Fig. 1-2A: same as Fig. 12A, but as a cross sectional profile;

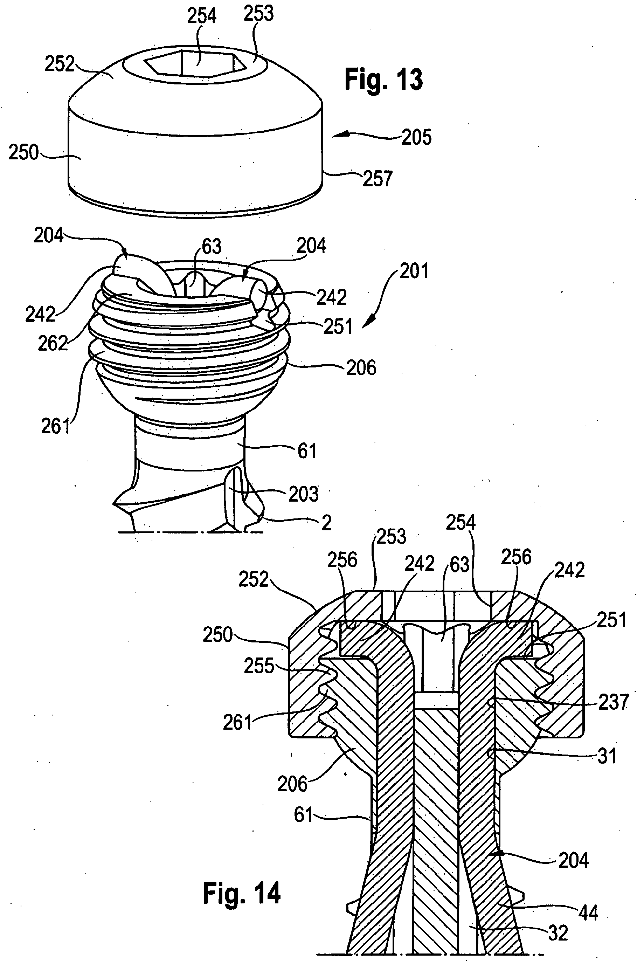

- Fig. 13: shows a perspective view of a head portion with locking cap of a bone anchor according to a third embodiment;
- Fig. 14: same as Fig. 13, but as a cross sectional view and in an assembled and expanded state of the pin-shaped elements;

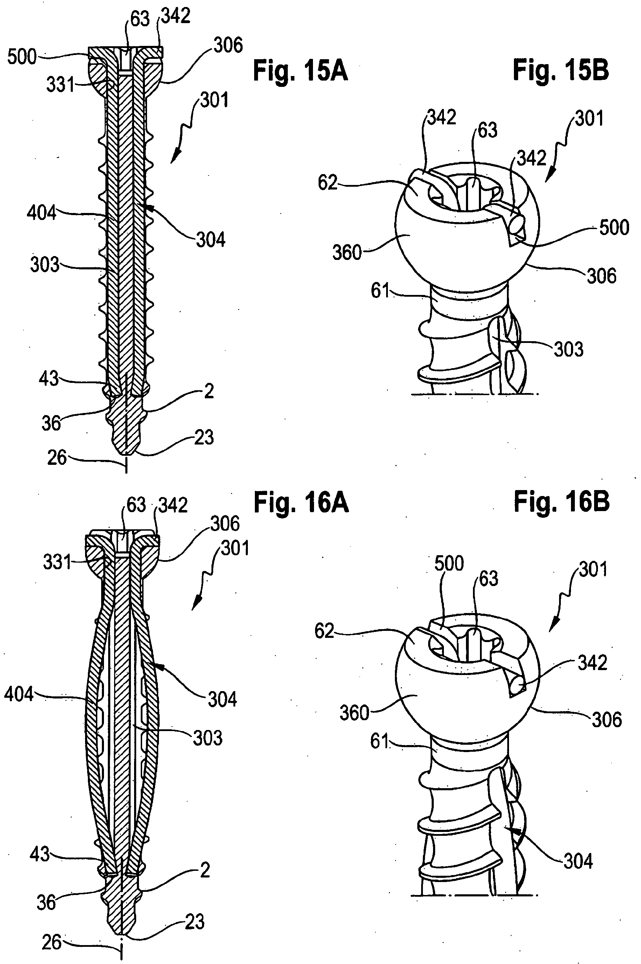

- Fig. 15A: shows a cross-sectional profile of the bone anchor and pin-shaped element according to a fourth embodiment in a first configuration.
- Fig. 15B: shows an enlarged perspective view of the head portion of the bone anchor of Fig. 15A.
- Fig. 16A: shows a cross-sectional profile of the bone anchor and pih-shaped element according to Fig. 15A in a second configuration.
- Fig. 16B: shows an enlarged perspective view of the head portion of the bone anchor of Fig. 16A.

A first embodiment of a bone anchoring assembly with a bone anchor according to the invention will be explained with regard to Figures 1 through 8C. The bone anchor assembly of this first embodiment comprises a bone anchor 1 and two pin-shaped elements 4, one of which is shown each in Figures 1 and 2.

The bone anchor 1 comprises a main body including a head 6 and a shank 2. The main body shown in the embodiments is a contiguous, monolithic body, but may generally also consist of multiple parts, wherein for example the head and the shank, or additionally the tip portion 23, are separate parts connectable to each other. The head 6 has a spherically segment-shaped contour 60, a neck portion 61 forming a transition to the shank 2 and a flat top face 62. In the top face, an engagement portion 63 is formed as a recess, which in this example is torx-shaped, but any other shape such as hexagonal socket or recess shape etc. is possible as well.

The shank 2 extends from the neck portion 61 up to the tip 23 and is of substantially cylindrical shape with a conical shape or a tapering towards the tip 23. A bone thread 22 extends along the entire length of the shank 2, wherein the thread 22 is formed by a helical crest 24 and a corresponding thread root 25 formed between respective crest 24 portions of each turn.

As can best be seen in Fig. 2, two recesses 3 extend each from one opening 37 formed at the bottom of the engagement portion 63 through a portion of the head 6 and its neck portion 61 along the shank 2 up to a bore hole 33 with a stop 36. The bore hole 33 and stop 36 are located near the tip 23 of the bone anchor 1, substantially where the tapering or narrowing towards the tip 23 starts. The opening 37 corresponds to a first end, and the stop 36 corresponds to a second end of the recess 3. Each of the two recesses 3 are configured to receive one of the pin-shaped elements 4 shown on the left sides each of Fig. and 2.

The recesses of this embodiment have an almost straight and linear shape except the bore hole 33 adjacent the second end, or stop 36, of each recess 3, which is slightly inclined towards a central longitudinal axis 26 of the main body of the bone anchor 1. More specifically, the recesses 3 extend substantially parallel to the central longitudinal axis 26 of the main body, and are arranged symmetrically and mutually opposite each other.

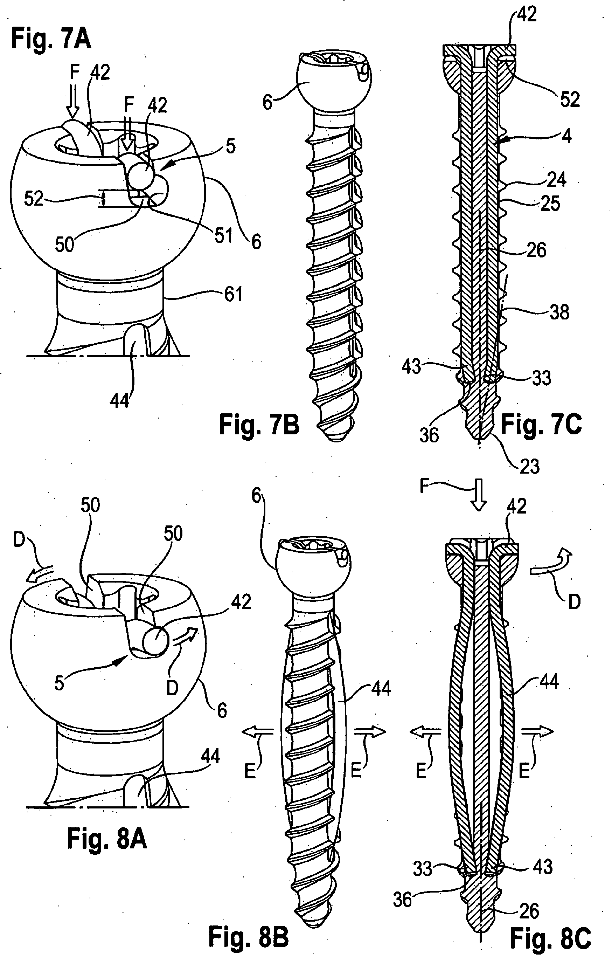

The recesses 3 according to the first embodiment comprise three portions: (a) a first portion formed as a bore or through hole 31 extending through the head 6 and neck portion 61, (b) a second portion formed as a groove portion 32 extending along a surface of the shank 2, and (c) a third portion corresponding to the above mentioned bore hole 33. Through hole 31 and bore hole 33 fully enclose a pin-shaped element 4 received therein, as can be seen for example in Figs. 7C and 8C, while the groove portion 32 is open in radial outward direction, as can best be seen in Fig. 1. Groove portion 32 thus interrupts the thread 22, and in particular each turn of crests 24 and roots 25. As will be explained below, the groove portion 32 allows the pin-shaped element 4 inserted in the recess 3 to bend and expand radially outwards within a range between respective openings 39a, 39b of through hole 31 and bore hole 33 facing each other.

The stop 36 is formed as a flat, rounded, conical, tapered or otherwise shaped face at the bottom of the bore hole 33 and is configured to receive and engage with the distal end portion 43 of the pin-shaped element, when it is inserted, and to exert a counterforce in axial direction (longitudinal axis 26), when the pin-shaped element 4 is compressed.

The pin-shaped element 4 has an elongated straight and linear shape wherein a proximal end portion 42 is kinked at a right angle with respect to a remainder portion 41 of the pin-shaped element. The recesses 3 of the bone anchor 1 have a width between the first and second ends, and a diameter of the pin-shaped element 4 is equal to or slightly smaller than said width such as to be received in the recess 3. The length of the pin-shaped element 4 is larger than the length of the recesses 3 between the first and second ends, or between the opening 37 and the stop 36, respectively, such that the pin-shaped element 4 protrudes from the opening 37 in an uncompressed, unbent or unbiased state.

Compression, bending and radial expansion of the pin-shaped element 4 is maintained by a locking structure 5, which will be explained in the following:

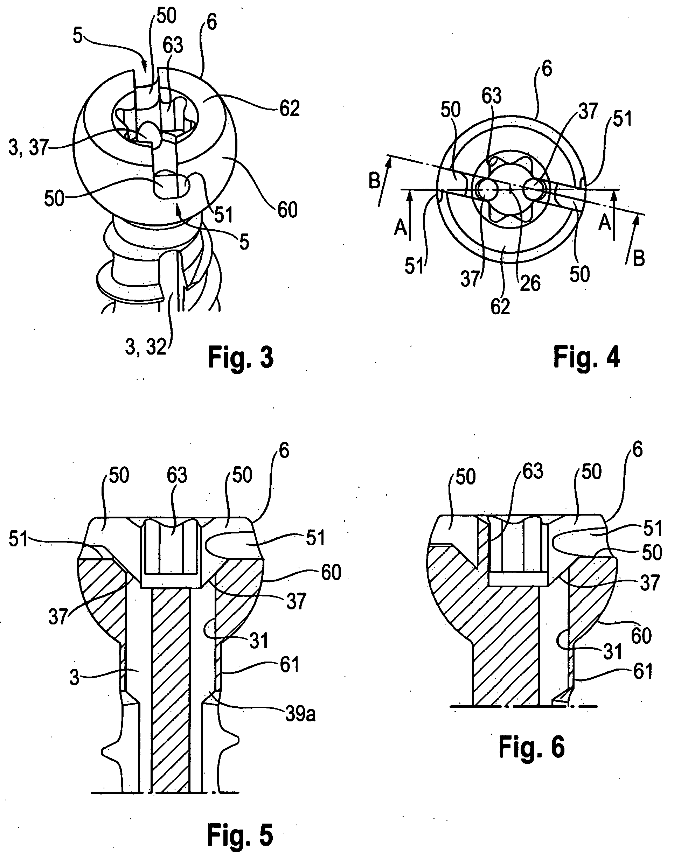

The embodiments as described herein mainly differ from each other by the respective mechanism of locking. Details of the locking structure 5 according to the first embodiment are depicted in Figures 3 through 6. The locking structure 5 of the first embodiment refers to a bayonet catch. It comprises each one channel 50 and a corresponding catching recess 51 adjacent to channel 50. Each one locking structure 5, or bayonet catch is provided for each recess 3. The channels 50 extend transverse to the longitudinal axis 26 of the main body, and as can be seen in Fig. 4 - extend adjacent to the opening 37 of the recess 3 such as to receive the proximal end portion 42 which protrudes from the opening and which is kinked at right angle at the pin-shaped element 4.

The openings 37 of respective recesses 3 are advantageously formed at the bottom of the engagement portion 63 within each one of the six mutually opposite lateral recesses of the torxshape. As a consequence, the two channels 50 which open adjacent the openings 37 cut a wall formed between the engagement portion 63 and the outer spherically segment-shaped contour 60 of the head 6. It is noted that the term "adjacent" as used in this document with regard to the locking structure does not necessarily mean that for example the opening and the locking structure contact each other or are contiguous. As small distance is possible. The distance should not extend a length of the engagement portion.

The catching recesses 51 extend laterally from a bottom portion of respective channels 50 such as to receive the proximal end portions 42 of the pin-shaped elements 4, when these are rotated in an azimuthal direction around the longitudinal axis 26. As shown in the top view of Fig. 4, the channels 50 have an axis BB and the catching recesses have an axis AA which is slightly rotated with respect to axis BB of the channels 50 around the central longitudinal axis 26 of the main body.

A length between the second end, or stop 36, of the recess 3 and an upper wall of the catching recess 51 measured along the longitudinal axis direction 26 is less than a length between respective ends of the pin-shaped element 4 including distal and proximal end portions 42,43, respectively. As a consequence, when the proximal end portion 42 is received in the catching recess 51, a pin-shaped element 4 is compressed and bent as can be seen in Figures 8B and 8C detailed below. The catching recess 51 preferably has an upper wall with a further recessed portion where the proximal end portion 42 may latch in. Such further recess portion may help to retain the proximal end portion 42 in the latched state. As common in the bayonet catch mechanism, further compression force and bending is then necessary to unlock the bent pin-shaped element 4.

The operation of compression and radial expansion is shown with reference to Figures 7A through 8C. Figs. 7A-7C show a first step wherein pin-shaped elements 4 are inserted into respective recesses 3 through openings 37 with proximal end portions 42 received in channels 50 when distal end portions 43 of the pin-shaped elements 4 abut on respective stops 36 at the second ends of the recesses 3. In this state, a distance 52 remains in the compression-free state between the proximal end portions 42 and a bottom wall of channels 50. The amount of bending depends on the value of this distance 52. In a next step (not shown) a tool (also not shown) is employed to apply a biasing force F onto proximal ends 42 of pin-shaped elements 4 in the longitudinal axis direction 26. Consequently, pin-shaped element 4 is further pressed against stop 36 at the distal end portion 43. As explained above, respective bore holes 33 include a central axis 38 indicated in Fig. 7C which is inclined with respect to the longitudinal axis 26 of the main body at an acute angle. Further insertion thus slightly bends the distal end portion 43 towards the central longitudinal axis 26 thereby predefining a radial bending direction E for an intermediate portion 44 of the pin-shaped element 4 as shown in Figs. 8B and 8C. Since the groove portion 32 is open, the intermediate portion 44 may leave the groove portion upon bending and expands radially outwards.

When the bone anchor 1 in this state has already been implanted in a bone, the intermediate portions 44 of both pin-shaped elements 4 advance into the surrounding bony material to improve and further support the anchoring stability of the bone anchor 1.

In a next step shown in Fig. 8A-8C, when the proximal end portion 42 has reached the bottom wall of the channel 50, the tool (not shown) is used to rotate the proximal end portions 42 in an azimuthal direction D to latch the same into the catch recesses 51. Once the proximal end portions 42 are latched into the catching recesses, the bending of the pin-shaped elements 4 is locked and maintained by the locking structure 5. Unlocking of the pin-shaped elements may be performed with the same steps in the reverse order.

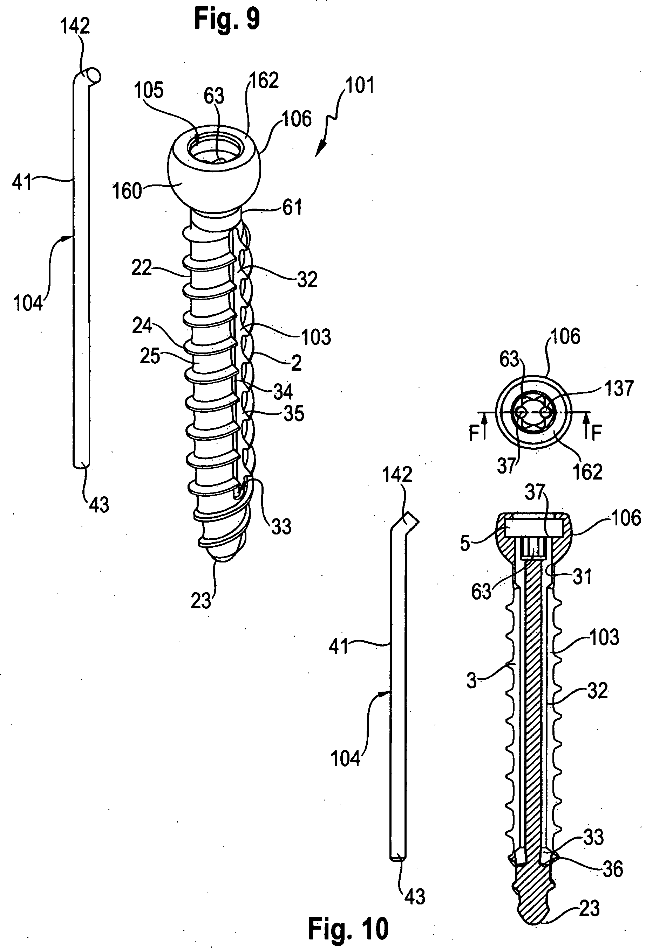

A second embodiment of a bone anchoring assembly is explained with reference to Figures 9 through 12B. An overview of the second embodiment of a bone anchor 101 is given in Figures 9 and 10, wherein like parts with respect to the first embodiment are denoted with the same reference numerals and repeated explanation thereof shall be avoided herein. This particularly refers to features of the shank 2 of the bone anchor 101. Differences arise with respect to the head 106. While the head 106 also has a spherically segment-shaped contour 160 and a flat annular top face 162, the locking structure 105 formed therein comprises features of an undercut 150 in order to lock the proximal end portions 142 of pin-shaped element 104.

As shown in Fig. 11A, which shows a first step of inserting the pin-shaped elements 104 into respective openings 137 of recesses 103, an annular opening 151 is formed in the top face 162, wherein the annular opening 151 has a diameter which is smaller than that of the undercut recess 150 adjacent below the opening 151. Accordingly, in a state where the bone anchor 101 has been implanted in the bone and the pin-shaped elements 104 are inserted into openings 137 providing first ends of respective recesses 103, a biasing force G has to be exerted onto pin-shaped elements 104 in order to bend their intermediate portions 44 in a radial direction E (shown in Fig. 12B).

The proximal end portions 142 of the pin-shaped elements 104 in this embodiment are not kinked at a right angle as in the first embodiment, but at a more obtuse angle, such that when these abut on an edge between the annular opening 151 and the planar top face 162, these proximal end portions 142 slidingly bend inwards (direction H in Fig. 11A), and finally latch into the undercut recess 150 as shown in Fig. 11B (direction J). The proximal end portions 142 are thus locked against an expansion in the axial direction (longitudinal axis 26) by an upper wall 152, or abutment face, of the undercut recess 150. As can be seen in Fig. 12B, the intermediate portion 44 of the pin-shaped element 4 is compressed and bent between the stop 36 adjacent the second end of the recess 103 and the upper wall 152 of the undercut recess 150 of locking structure 150 adjacent the opening 137.

A third embodiment will be described with reference to Figures 13 and 14. Like parts are denoted with the same reference numerals as in the previous embodiments and repeated explanation shall be avoided herein.

As can be seen in Fig. 13, the bone anchor 201 comprises a main body with a shank 2 and a head 206, wherein the shank 2 is similar to that of the previous embodiments. The head 206 has an external thread 261 which is provided to receive a corresponding inner thread 255 of a locking cap 257. The locking cap 257 and respective channels 251, which receive proximal end portions 242 kinked at right angle at one end of respective pin-shaped elements 204 form the locking structure 205 of the third embodiment. The channels 251 are formed transverse to the central longitudinal axis 26 of the main body and cut through a wall.

The locking cap 257 comprises a substantially cylindrical outer surface 250 and a spherical top face 252 with a flat centre face 253, in which a hexagon-shaped engagement portion 254 for engagement with an external tool is formed.

Fig. 14 shows the head portion in a state where the locking cap is attached and locks the proximal end portions 242 of the pin-shaped elements. In this state, the intermediate portions 44 of the pin-shaped elements 204 are expanded.

As can be seen in Fig. 13, when the pin-shaped elements 204 are inserted into respective recesses 203 of the bone anchor 201, end portions 242 protrude from openings 237 of the recesses 203 and even from the channels 251 above the flat top face 262, when the distal end portions 43 abut on the stops 36.

As can be seen in Fig. 14, a biasing force is exerted onto the proximal end portions 242 in an axial direction, i.e., along the longitudinal axis 26 of the bone anchor 201. More specifically, upon screwing the locking cap 257 with its inner thread 255 onto the outer thread 261 of the head 206, an engagement surface 256 provided in an inner space of the locking cap 257 engages an upper face of the proximal end portions 242 and presses the same down. As a consequence, like in the previous embodiments, the distal end portions 43 of the pin-shaped elements 204 abut on the stop 36 adjacent the second end of the recesses 203 which then exert a counterforce compressing the pin-shape element, such that the intermediate portions 44 bend radially outwards. Simultaneously, an extent of the pin-shaped element measured along the axis 26 shrinks and the proximal end portions 242 are pressed and move into the channels 251.

It is not necessary that the end portions 242 are kinked at a right angle. The locking cap may, for example, further bend the proximal end portions 242, when these are already only slightly kinked, further downwards upon locking.

The same or similar effect may be achieved as described with respect to the previous embodiments. Nevertheless, the third embodiment involves an additional part, i.e. the locking cap 257, which avoids latching mechanisms as provided in the first and second embodiments. Installation and removal of the bone anchor 203 may thus be facilitated. Only a common screw driver is needed.

A fourth embodiment will be described with reference to Figures 15A to 16B. Like parts are denoted with the same reference numerals as in the previous embodiments and repeated explanation shall be avoided herein.

As can be seen in Fig. 15A, the bone anchor 301 comprises a main body with a shank 2 and a head 306, wherein the shank 2 is similar to that of the previous embodiments. The head 306 has a spherically segment-shaped contour 360, a neck portion 61 forming a transition to the shank 2 and a flat top face 62. In the top face, an engagement portion 63 is formed as a recess as in the previous embodiments. The recesses 303 for receiving the pin-shaped element 304 are shaped as in the first embodiment. In the head 306 a groove 500 is formed that extends in a transverse direction to the longitudinal axis 26 of the main body and that has a depth that is greater than a thickness of the proximal end portion 342 of the pin-shaped element 304. The groove 500 is configured to receive the end portion 342 of the pin-shaped element 304.

The pin-shaped element 304 comprises the kinked proximal end portion 342, an intermediate portion 404 and a distal end portion 43. In this embodiment, the pin-shaped element 304 has shape memory properties and can assume a first configuration at a first temperature in which the intermediate portion 404 is substantially straight and in which the pin-shaped element 304 is insertable into the longitudinal recess 303 and movable towards the stop 36 of the longitudinal recess 303. In the first configuration, the length of the intermediate portion 404 is such that when the pin-shaped element 304 is inserted into the longitudinal recess 303, and the distal end portion abuts against the stop 36. The lower side of the proximal end portion 342 has a distance from the bottom of the groove 500 as can be seen in Fig. 15B. The pin-shaped element 304 can further assume a second configuration at a second temperature, that is higher than the first temperature, wherein in the second configuration the intermediate portion 404 of the pin-shaped element is bent in a transverse direction away from the shank 2. In the second configuration, when the distal end portion 43 abuts against the stop 36, the total length of the pin-shaped element 304 in an axial direction is reduced, such that the lower side of the proximal end portion 342 rests on the bottom of the groove 500, as can be seen in Figs. 16A and 16B.

The pin-shaped element 304 may be configured such that the transformation from the first configuration to the second configuration takes place when the pin-shaped element 304 is heated from the first temperature, that may be room temperature, to the second temperature, that may be body temperature.

In use, the bone anchoring assembly consisting of the bone anchor 301 and the pin-shaped element 304 is preassembled, wherein the pin-shaped element 304 is in the first configuration. Then the bone anchor is inserted into the bone. Through heating to the body temperature the pin-shaped element 304 can assume the second configuration in which the intermediate portion 404 is bent in a transverse direction away from the shank. The heating step can be performed using body heat or using an separate heating device.

The pin-shaped elements 4, 104, 204 of the first to third embodiments are preferably made from a flexible wire material, such as Kirschner wire, such as stainless steel, titanium alloys or other suitable, bio compatible materials. Sufficient bending flexibility is achieved by a diameter of 1 mm or less, preferably 0,75 mm or less, or more preferably 0,5 mm or less. The pin-shaped element 304 of the fourth embodiment is made preferably of Nitinol but other shape memory materials can also be used. These could be for example other metal alloys or plastic materials exhibiting a shape memory effect.

Materials for the main bodies of bone anchors employed for these or other embodiments can be taken from bio compatible materials including metals such as titanium, titanium alloys, nitinol, stainless steel, or plastic materials including PEEK, PCU, or similar materials.

The application field of the bone anchors described in these and other embodiments is not restricted to a treatment of fractures or osteoporosis, or to trauma surgery. For example, specific applications in the field of the vertebra column may also be envisaged.

In the above embodiments, bone anchors with shanks having a bone thread are shown. However, other types of shanks having thread less surfaces or being formed with barb elements may also be used.

In the above embodiments, spherically segment-shaped heads of bone anchors are described. However, any other shape of heads are possible. For example cylindrical, conical, etc. Further embodiments encompass bone anchors, in which no dedicated head is provided. For example, an end portion of the shank includes an engagement portion, which whereby defines a head portion.

In the above embodiments, the recess configured to receive the pin-shaped elements is described to have a substantially straight shape. However, it is also possible that the recesses extend helically around the shank portion.

In the above embodiments, the pin-shaped elements are described to have a round cross sectional profile. However, triangular, square or other profiles are possible as well, for example strip-like profiles. Additionally, the pin-shaped elements can be made from plastic material. In this case, however, fatigue breakage or damage has to be considered here.

In the above embodiments an inclined bore hole 33 having a stop 36 is provided at a second end of the recess receiving the pin-shaped element. However, the bore hole may not need to be inclined, and further, the bore hole needs not to have a constant diameter, but can have a conical or any other profile like being tapered towards the distal end. Still further, the stop 36 needs not can refer to a clamping means firmly holding the second distal end portion of the pin-shaped element.

In the above embodiments, proximal end portions are kinked at an angle with respect to a main portion of the pin-shaped elements. However, other embodiments include straight, non-kinked end portions and the locking structure presses on an end face or tip of the proximal end portion.

## Claims

1. A bone anchor (1, 101, 201), comprising:
a main body defining a longitudinal axis (26) and comprising:
a head (6, 106, 206); and
a shank (2);
at least one longitudinal recess (3, 103, 203) having a first end formed at the head (6, 106, 206) and a second end formed at the shank (2), the recess (3, 103, 203) extending from the first end through a portion of the head (6, 106, 206) and along a portion of the shank (2) to the second end, wherein the recess (3, 103, 203) is configured to receive therein a pin-shaped element (4, 104, 204);
a stop (36) being provided at the second end of the recess (3, 103, 203), said stop (36) being configured to engage or abut a distal end portion (43) of the pin-shaped element (4, 104, 204);
a locking structure (5, 105, 205) being provided adjacent the first end of the recess (3, 103, 203), said locking structure (5, 105, 205) being configured to engage a proximal end portion (42, 142, 242) of the pin-shaped element (4, 104, 204) and exert a biasing force thereon towards the stop (36) such as to stress and bend an intermediate portion (44) of the pin-shaped element (4, 104, 204) in a transverse direction (E) away from the shank (2).

2. The bone anchor (1, 101, 201) of claim 1, wherein
the portion of the recess extending through the head is formed as a through hole (31), wherein the first end of the recess (3, 103, 203) is provided as an opening (37, 137, 237) in the head.

3. The bone anchor (1, 101, 201) according to one of claims 1 to 2, wherein
at least a part of the portion of the recess (3, 103, 203) extending along the shank (2) is formed as a groove portion (32) being open towards the outside of the bone anchor to allow the pin-shaped element (4, 104, 204), when being inserted, to bend away from the shank (2).

4. The bone anchor (1, 101, 201) according to claim 3, wherein
a depth of the groove portion is equal to or larger than the width such that a pin-shaped element (4, 104, 204) being received in the groove portion (32) does not protrude from an outer surface of the main body, when the pin-shaped element (4, 104, 204) is received therein but not yet stressed and bent by the locking structure (5, 105, 205).

5. The bone anchor (1, 101, 201) according to one of claims 1 to 4, comprising
two of said recesses (3, 103, 203) which are arranged on mutually opposite sides of the bone anchor (1, 101, 201).

6. The bone anchor (1, 101, 201) according to one of claims 1 to 5, wherein
the at least one recess (3, 103, 203) has, excluding a portion adjacent the second end, a straight shape.

7. The bone anchor (1, 101, 201) according to claim 6, wherein
the at least one recess (3, 103, 203) is, excluding a portion adjacent the second end, parallel to the longitudinal axis (26).

8. The bone anchor (1, 101, 201) according to one of claims 1 to 7, wherein:
a portion of the at least one recess (3, 103, 203) adjacent to the second end is provided as a bore hole (33) in order to hold the distal end portion (43) of the pin-shaped element (4, 104, 204) in position during stressing and bending.

9. The bone anchor (1, 101, 201) according to claim 8, wherein
the bore hole (33) has an axis (38) inclined with respect to and oriented towards the longitudinal axis (26) to facilitate bending away of the intermediate portion (44) from the longitudinal axis (26) upon exertion of the biasing force.

10. The bone anchor (1, 101, 201) according to one of claims 2 to 9, wherein
the head (6, 106, 206) of the bone anchor has a recessed engagement portion (63) for engagement with a tool, wherein the first end of the at least one recess (3, 103, 203) being provided as an opening (37, 137, 237) opens into the recessed engagement portion (63).

11. A bone anchoring assembly,
comprising the bone anchor (1, 101, 201) according to one of claims 1 to 10; and
the pin-shaped element (4, 104, 204), wherein the proximal end portion (42, 142, 143) is kinked at a right or an oblique angle.

12. The bone anchoring assembly of claim 11,
wherein the locking structure (5) is formed as a bayonet catch configured to receive the kinked proximal end portion (42) when the intermediate portion (44) of the pin-shaped element (4) is in a stressed and bent state.

13. The bone anchoring assembly of claim 12,
wherein the locking structure (5) comprises:
a channel (50) formed in the head (6) adjacent the first end of the recess (3), said channel (50) having an axis (BB) transverse to the longitudinal axis (26), said channel (50) being configured to guide therethrough the kinked proximal end portion (42) of the pin-shaped element (4); and
a catching recess (51) adjacent to and laterally opening into the channel (50) such as to receive the kinked proximal end portion (42) from the channel (50).

14. The bone anchoring assembly of claim 11,
wherein the locking structure (105) comprises:
an undercut recess (150) formed in the head (106) adjacent to the first end of the recess (103), wherein the undercut recess (150) is configured to receive the kinked proximal end portion (142) when the intermediate portion (44) is stressed and bent.

15. The bone anchoring assembly of claim 11,
wherein the locking structure (205) comprises:
a locking cap (257), which may be attached to the head of the bone anchor, and which comprises an abutment face (256) which exerts a biasing force upon the kinked proximal end portion (242) to stress and bend the intermediate portion (44) of the pin-shaped element (204).

16. The bone anchoring assembly of claim 15, wherein:
the locking cap (205) has an internal thread (255) mating with an external thread (261) provided at the head (206) of the bone anchor to facilitate attachment of the locking cap (257) to the head (206).

## Patentansprüche

1. Ein Knochenanker (1, 101, 201), umfassend:
einen Hauptkörper, der eine Längsachse (26) festlegt und umfasst:
einen Kopf (6, 106, 206); und
einen Schaft (2);
wenigstens eine Längsausnehmung (3, 103, 203) mit einem an dem Kopf (6, 106, 206) ausgebildeten ersten Ende und einem an dem Schaft (2) ausgebildeten zweiten Ende, wobei sich die Ausnehmung (3, 103, 203) von dem ersten Ende durch einen Abschnitt des Kopfs (6, 106, 206) und entlang eines Abschnitts des Schafts (2) bis zu dem zweiten Ende erstreckt, wobei die Ausnehmung (3, 103, 203) dazu eingerichtet ist, darin ein stiftförmiges Element (4, 104, 204) aufzunehmen;
einen Anschlag (36), der an dem zweiten Ende der Ausnehmung (3, 103, 203) vorgesehen ist, wobei der Anschlag (36) eingerichtet ist, mit einem distalen Endabschnitt (43) des stiftförmigen Elements (4, 104, 204) in Eingriff zu treten oder an dieses anzustoßen;
Eine Verschlussstruktur (5, 105, 205), die benachbart zu dem ersten Ende der Ausnehmung (3, 103, 203) vorgesehen ist, wobei die Verschlussstruktur (5, 105, 205) eingerichtet ist, mit einem proximalen Endabschnitt (42, 142, 242) des stiftförmigen Elements (4, 104, 204) in Eingriff zu treten und eine Spannkraft auf dieses in Richtung auf den Anschlag (36) auszuüben, um einen mittleren Abschnitt (44) des stiftförmigen Elements (4, 104, 204) in einer Querrichtung (E) weg von dem Schaft (2) zu spannen und zu biegen.

2. Der Knochenanker (1, 101, 201) gemäß Anspruch 1, wobei
der Abschnitt der Ausnehmung, der sich durch den Kopf erstreckt, als ein Durchtrittsloch (31) ausgebildet ist, wobei das erste Ende der Ausnehmung (3, 103, 203) als eine Öffnung (37, 137, 237) in dem Kopf vorgesehen ist.

3. Der Knochenanker (1, 101, 201) gemäß einem der Ansprüche 1 bis 2, wobei
zumindest ein Teil des Abschnitts der Ausnehmung (3, 103, 203), der sich entlang dem Schaft (2) erstreckt, als ein Nutabschnitt (32) ausgebildet ist, der in eine Richtung nach außerhalb des Knochenankers offen ist, um es dem stiftförmigen Element (4, 104, 204) zu ermöglichen, wenn es eingesetzt ist, sich weg von dem Schaft (2) zu biegen.

4. Der Knochenanker (1, 101, 201) gemäß Anspruch 3, wobei
eine Tiefe des Nutabschnitts gleich oder größer ist als seine Breite, so dass ein stiftförmiges Element (4, 104, 204), das in dem Nutabschnitt (32) aufgenommen ist, nicht von einer Außenoberfläche des Hauptkörpers hervorsteht, wenn das stiftförmige Element (4, 104, 204) darin aufgenommen aber noch nicht durch die Verschlussstruktur (5, 105, 205) gespannt und gebogen ist.

5. Der Knochenanker (1, 101, 201) gemäß einem der Ansprüche 1 bis 4, umfassend:
zwei der Ausnehmungen (3, 103, 203), die auf zueinander entgegengesetzten Seiten des Knochenankers (1, 101, 201) angeordnet sind.

6. Der Knochenanker (1, 101, 201) gemäß einem der Ansprüche 1 bis 5, wobei
die wenigstens eine Ausnehmung (3, 103, 203) ausgenommen einen Abschnitt benachbart zu dem zweiten Ende eine gerade Form besitzt.

7. Der Knochenanker (1, 101, 201) gemäß Anspruch 6, wobei
die wenigstens eine Ausnehmung (3, 103, 203) ausgenommen einen Abschnitt benachbart zu dem zweiten Ende parallel zur Längsachse (26) ist.

8. Der Knochenanker (1, 101, 201) gemäß einem der Ansprüche 1 bis 7, wobei:
ein Abschnitt der wenigstens einen Ausnehmung (3, 103, 203) benachbart zu dem zweiten Ende als ein Bohrungsloch (33) vorgesehen ist, um den distalen Endabschnitt (43) des stiftförmigen Elements (4, 104, 204) während des Spannens und Biegens in Position zu halten.

9. Der Knochenanker (1, 101, 201) gemäß Anspruch 8, wobei
das Bohrungsloch (33) eine Achse (38) besitzt, die in Bezug auf die Längsachse (26) geneigt und zu dieser hin orientiert ist, um das Wegbiegen des mittleren Abschnitts (44) von der Längsachse (26) beim Ausüben der Spannkraft zu bewirken.

10. Der Knochenanker (1, 101, 201) gemäß einem der Ansprüche 2 bis 9, wobei der Kopf (6, 106, 206) des Knochenankers einen ausgesparten Eingriffsabschnitt (63) zum Eingriff durch ein Werkzeug besitzt, wobei das erste Ende der wenigstens einen Ausnehmung (3, 103, 203), die als eine Öffnung (37, 137, 237) vorgesehen ist, sich in den ausgesparten Eingriffsabschnitt (63) erstreckt.

11. Eine Knochenverankerungsanordnung,
umfassend den Knochenanker (1, 101, 201) gemäß einem der Ansprüche 1 bis 10; und
das stiftförmige Element (4, 104, 204), wobei der proximale Endabschnitt (42, 142, 143) um einen rechten oder einen geneigten Winkel abgeknickt ist.

12. Die Knochenverankerungsanordnung gemäß Anspruch 11,
wobei die Verschlussstruktur (5) als ein Bajonettverschluss ausgebildet ist, der eingerichtet ist, den abgeknickten proximalen Endabschnitt (42) aufzunehmen, wenn sich der mittlere Abschnitt (44) des stiftförmigen Elements (4) in einem belasteten und gebogenen Zustand befindet.

13. Die Knochenverankerungsanordnung gemäß Anspruch 12,
wobei die Verschlussstruktur (5) umfasst:
einen Kanal (50), der in dem Kopf (6) benachbart zu dem ersten Ende der Ausnehmung (3) ausgebildet ist, wobei der Kanal (50) eine Achse (BB) quer zur Längsachse (26) besitzt, wobei der Kanal (50) eingerichtet ist, den abgeknickten proximalen Endabschnitt (42) des stiftförmigen Elements (4) durch sich zu führen; und
eine Arretierungsausnehmung (51) benachbart zu dem Kanal (50) und sich seitlich in den Kanal (50) öffnend, um den abgeknickten proximalen Endabschnitt (42) von dem Kanal (50) aus aufzunehmen.

14. Die Knochenverankerungsanordnung gemäß Anspruch 11,
wobei die Verschlussstruktur (105) umfasst:
eine Hinterschnittausnehmung (150), die in dem Kopf (106) benachbart dem ersten Ende der Ausnehmung (103) ausgebildet ist, wobei die Hinterschnittausnehmung (150) eingerichtet ist, den abgeknickten proximalen Endabschnitt (142) aufzunehmen, wenn der mittlere Abschnitt (44) belastet und gebogen ist.

15. Die Knochenverankerungsanordnung gemäß Anspruch 11,
wobei die Verschlussstruktur (205) umfasst:
eine Verschlusskappe (257), die an dem Kopf des Knochenanker angebracht werden kann, und die eine Anschlagfläche (256) umfasst, die eine Spannkraft auf den abgeknickten proximalen Endabschnitt (242) ausübt, um den mittleren Abschnitt (44) des stiftförmigen Elements (204) zu spannen und zu biegen.

16. Die Knochenverankerungsanordnung gemäß Anspruch 15, wobei:
die Verschlusskappe (205) ein Innengewinde (255) besitzt, das zu einem am Kopf (206) des Knochenankers vorgesehenen Außengewinde (261) passt, um ein Anbringen der Verschlusskappe (257) an dem Kopf (206) zu ermöglichen.

## Revendications

1. Ancrage osseux (1, 101, 201), comprenant :
un corps principal définissant un axe longitudinal (26) et comprenant :
une tête (6, 106, 206) ; et
une tige (2) ;
au moins un retrait longitudinal (3, 103, 203) ayant une première extrémité formée au niveau de la tête (6, 106, 206) et une deuxième extrémité formée au niveau de la tige (2), le retrait (3, 103, 203), s'étendant depuis la première extrémité à travers une portion de la tête (6, 106, 206) et le long d'une portion de la tige (2) jusqu'à la deuxième extrémité, le retrait (3, 103, 203) étant configuré pour recevoir un élément en forme de broche (4, 104, 204) ;
une butée (36) étant prévue au niveau de la deuxième extrémité du retrait (3, 103, 203), ladite butée (36) étant configurée pour s'engager avec ou pour butter contre une portion d'extrémité distale (43) de l'élément en forme de broche (4, 104, 204) ;
une structure de verrouillage (5, 105, 205) étant prévue en position adjacente à la première extrémité du retrait (3, 103, 203), ladite structure de verrouillage (5, 105, 205) étant configurée pour s'engager avec une portion d'extrémité proximale (42, 142, 242) de l'élément en forme de broche (4, 104, 204) et pour exercer une force de sollicitation sur celle-ci en direction de la butée (36) de manière à solliciter et faire fléchir une portion intermédiaire (44) de l'élément en forme de broche (4, 104, 204) dans une direction transversale (E) à l'écart de la tige (2).

2. Ancrage osseux (1, 101, 201) selon la revendication 1, dans lequel
la portion du retrait s'étendant à travers la tête est formée en tant que trou traversant (31), la première extrémité du retrait (3, 103, 203) étant prévue en tant qu'ouverture (37, 137, 237) dans la tête.

3. Ancrage osseux (1, 101, 201) selon l'une quelconque des revendications 1 à 2, dans lequel au moins une partie de la portion du retrait (3, 103, 203) s'étendant le long de la tige (2) est formée en tant que portion de rainure (32) ouverte vers l'extérieur de l'ancrage osseux pour permettre à l'élément en forme de broche (4, 104, 204), lorsqu'il est inséré, de fléchir à l'écart de la tige (2).

4. Ancrage osseux (1, 101, 201) selon la revendication 3, dans lequel
une profondeur de la portion de rainure est supérieure ou égale à la largeur de telle sorte qu'un élément en forme de broche (4, 104, 204) reçu dans la portion de rainure (32) ne fasse pas saillie depuis une surface extérieure du corps principal lorsque l'élément en forme de broche (4, 104, 204) est reçu à l'intérieur mais n'est pas encore sollicité et fléchi par la structure de verrouillage (5, 105, 205).

5. Ancrage osseux (1, 101, 201) selon l'une quelconque des revendications 1 à 4, comprenant deux desdits retraits (3, 103, 203) qui sont disposés sur des côtés mutuellement opposés de l'ancrage osseux (1, 101, 201).

6. Ancrage osseux (1, 101, 201) selon l'une quelconque des revendications 1 à 5, dans lequel l'au moins un retrait (3, 103, 203) présente, à l'exception d'une portion adjacente à la deuxième unité, une forme droite.

7. Ancrage osseux (1, 101, 201) selon la revendication 6, dans lequel
l'au moins un retrait (3, 103, 203), à l'exception d'une portion adjacente à la deuxième extrémité, est parallèle à l'axe longitudinal (26).

8. Ancrage osseux (1, 101, 201) selon l'une quelconque des revendications 1 à 7, dans lequel :
une portion de l'au moins un retrait (3, 103, 203) adjacente à la deuxième extrémité est prévue sous forme de trou d'alésage (33) afin de retenir la portion d'extrémité distale (43) de l'élément en forme de broche (4, 104, 204) en position au cours de sa sollicitation et de sa flexion.

9. Ancrage osseux (1, 101, 201) selon la revendication 8, dans lequel
le trou d'alésage (33) présente un axe (38) incliné par rapport à l'axe longitudinal (26) et orienté vers celui-ci afin de faciliter la flexion de la portion intermédiaire (44) à l'écart de l'axe longitudinal (26) lors de l'application de la force de sollicitation.

10. Ancrage osseux (1, 101, 201) selon l'une quelconque des revendications 2 à 9, dans lequel la tête (6, 106, 206) de l'ancrage osseux présente une portion d'engagement en retrait (63) pour l'engagement avec un outil, la première extrémité de l'au moins un retrait (3, 103, 203), prévue sous forme d'ouverture (37, 137, 237), s'ouvrant dans la portion d'engagement en retrait (63).

11. Ensemble d'ancrage osseux,
comprenant l'ancrage osseux (1, 101, 201) selon l'une quelconque des revendications 1 à 10 ; et l'élément en forme de broche (4, 104, 204), la portion d'extrémité proximale (42, 142, 143) étant pliée à angle droit ou suivant un angle oblique.

12. Ensemble d'ancrage osseux selon la revendication 11,
dans lequel la structure de verrouillage (5) est formée en tant que loquet à baïonnette configuré pour recevoir la portion d'extrémité proximale pliée (42) lorsque la portion intermédiaire (44) de l'élément en forme de broche (4) est dans un état sollicité et fléchi.

13. Ensemble d'ancrage osseux selon la revendication 12,
dans lequel la structure de verrouillage (5) comprend :
un canal (50) formé dans la tête (60) en position adjacente à la première extrémité du retrait (3), ledit canal (50) ayant un axe (BB) transversal à l'axe longitudinal (26), ledit canal (50) étant configuré pour guider à travers lui la portion d'extrémité proximale pliée (42) de l'élément en forme de broche (4) ; et
un retrait d'accrochage (51) adjacent au canal (50) et s'ouvrant latéralement dans celui-ci de manière à recevoir la portion d'extrémité proximale pliée (42) provenant du canal (50).

14. Ensemble d'ancrage osseux selon la revendication 11,
dans lequel la structure de verrouillage (105) comprend :
un retrait en contre-dépouille (150) formé dans la tête (106) en position adjacente à la première extrémité du retrait (103), le retrait en contre-dépouille (150) étant configuré pour recevoir la portion d'extrémité proximale pliée (142) lorsque la portion intermédiaire (44) est sollicitée et fléchie.

15. Ensemble d'ancrage osseux selon la revendication 11,
dans lequel la structure de verrouillage (205) comprend :
un capuchon de verrouillage (257), qui peut être attaché à la tête de l'ancrage osseux et qui comprend une face de butée (256) qui exerce une force de sollicitation sur la portion d'extrémité proximale pliée (242) pour solliciter et faire fléchir la portion intermédiaire (44) de l'élément en forme de broche (204).

16. Ensemble d'ancrage osseux selon la revendication 15, dans lequel :
le capuchon de verrouillage (205) présente un filetage interne (255) s'accouplant avec un filetage externe (261) prévu au niveau de la tête (206) de l'ancrage osseux pour faciliter la fixation du capuchon de verrouillage (257) à la tête (206).
